# EUROPEAN PATENT APPLICATION

(11) **EP 4 585 606 A1**
(43) Date of publication of application: **16.07.2025**
(21) Application number: 22961677.6
(22) Date of filing: 11.10.2022
(51) Int. Cl.: C07K 2/00, A61K 31/00, A61K 38/00, A61P 19/08

(54) **OLIGOPEPTIDE AND USE AND COMPOSITION THEREOF**

(71) Applicant: Buddhist Tzu Chi Medical Foundation, Hualien, Taiwan (TW)
(72) Inventor: HO, Tsung-Jung, Hualien, Taiwan (TW); LIN, Jung-Hsing, Hualien, Taiwan (TW); LIN, Shinn-Zong, Hualien, Taiwan (TW); TSAI, Wan-Ting, Hualien, Taiwan (TW); WU, Jia-Ru, Hualien, Taiwan (TW); CHEN, Hao-Ping, Hualien, Taiwan (TW)
(74) Representative: Manitz Finsterwald Patent- und Rechtsanwaltspartnerschaft mbB
(86) International application number: PCT/CN2022/124643
(87) International publication number: WO 2024/077483

(57) **Abstract**

Provided is an oligopeptide including 2 to 10 amino acids, and the oligopeptide includes an amino acid sequence of tyrosine-arginine (Tyr-Arg) and/or threonine-serine-lysine (Thr-Ser-Lys). Further provided in the present disclosure is use of the oligopeptide in manufacture of a medicament for treating or preventing bone-related diseases and/or conditions, including administering a therapeutically effective amount of the oligopeptide to a subject in need thereof. In addition, also provided in the present disclosure is an oligopeptide composition including the oligopeptide and a pharmaceutically acceptable carrier.

## Description

### CROSS REFERENCE TO RELATED APPLICATIONS

This application is a U.S. National Stage Application filed under 35 U.S.C. § 371, based on International Patent Application No. PCT/CN2022/124643, filed on October 11, 2022. The entire contents of the above application are incorporated herein by reference.

### BACKGROUND

### 1. Technical Field

The present disclosure relates to an oligopeptide, the use thereof for manufacture of a medicament for treating or preventing bone-related diseases and/or conditions, and a composition containing the same.

### SEQUENCE LISTING

The present disclosure is being filed along with a Sequence Listing in electronic format. The Sequence Listing is provided as a file entitled 80113US-Sequence Listing.XML, created on February 25, 2025, which is 14.4 kb in size. The information in the electronic format of Sequence Listing is incorporated herein by reference in its entirety.

### 2. Description of Associated Art

The bone density of humans begins to decrease year by year at the age of 30, and in particular, postmenopausal women or men at age of 65 or above are at high risk of developing osteoporosis, with a significantly higher proportion in women than in men for suffering from osteoporosis. Osteoporosis is a common chronic disease in elders at age of 65 or above, and recent studies have shown that the incidence of hip fracture in Taiwan, China is the highest in Asia.

The term osteoporosis refers to the formation of many voids in bones, which results in a hollow and brittle bone structure, compression fractures of the spine, hunchback, affecting height, etc., or causes injuries such as hard fractures and bone fractures when being subjected to external force collisions or falling. For many elders who have been developed osteoporosis, they often have to stay in bed for a long time or rely on others for care once they have a hip fracture. In statistics, about 20% of patients with hip fractures die within one year.

The medicaments currently used for treating osteoporosis in clinic includes calcitonin, hormone therapy and bisphosphonates, parathyroid hormone, etc., however, the medicaments described above can only be used in a patient who has been diagnosed with osteoporosis, i.e., the medicaments described above are forbidden if the bone loss does not meet the standard for osteoporosis.

On other hand, a long-term use of an osteoporosis therapeutic has an insufficient effect of the calcium enhancing material, and can result in side effects including breast cancer, myocardial infarction, venous thrombosis, etc. In addition, since calcium ions can initiate the critical signal for proliferation and differentiation of osteoblastic cells during bone remodeling, calcium supplements for enhancing bone care have been emerged continuously, however, the absorption of calcium depends on various factors, such as phosphoric acid, phytic acid, estrogen, fibroblast growth factor, parathyroid hormone, insulin-like growth factor-1, and calcitriol (vitamin D), etc., therefore the calcium taken from externals cannot be absorbed and utilized completely through intestinal tract.

As mentioned above, there is currently a need for providing a health product or medication that can effectively treat and/or prevent bone-related diseases and/or conditions (especially bone loss) without any side effects.

### SUMMARY

In order to solve the problem described above, the present disclosure provides an oligopeptide having the amino acid sequence of YR and/or TSK, which is ease in enteral absorption and increase the bioavailability of calcium by a person. Specifically, the oligopeptide of the present disclosure can promote the proliferation, differentiation and activation of osteoblastic cells and cartilage cells, and the oligopeptide and calcium ions have synergistic effects on the proliferation, differentiation and activation of osteoblasts, which can effectively prevent or treat bone degenerative diseases such as osteoporosis, arthritis deformans, etc.

Specifically, the present disclosure provides an isolated or synthetic oligopeptide comprising 2 to 10 amino acids, wherein the oligopeptide comprises an amino acid sequence of Tyr-Arg and/or Thr-Ser-Lys.

In an embodiment, the oligopeptide comprises at least one amino acid sequence selected from Thr-Ser-Lys-Tyr-Arg (TSKYR, SEQ ID No. 1), Thr-Ser-Lys-Tyr (TSKY, SEQ ID No. 2), Thr-Ser-Lys (TSK), and Tyr-Arg (YR).

In an embodiment, the oligopeptide promotes a proliferation and differentiation of osteoblastic cells and/or cartilage cells.

In an embodiment, the oligopeptide increases an expression of at least one gene selected from the group consisting of type II collagen (COL2A1), aggrecan (ACAN), RUNX2, OCN, FGFR2, and FGFR3.

In addition, the oligopeptide of the present disclosure can be prepared by a hydrolysis process, an extraction process, a fermentation process, a synthesis process, a generic recombination engineering process, or a combination thereof. In an embodiment, the oligopeptide is prepared by the hydrolysis process of a Traditional Chinese medicine or by the synthesis process directly.

The present disclosure provides a use of the oligopeptide in manufacture of a medicament for treating or preventing bone-related diseases and/or conditions, comprising administering a therapeutically effective amount of the oligopeptide to a subject in need thereof.

In an embodiment, the bone-related diseases and/or conditions include osteoarthritis, bone loss, arthritis, osteoporosis, fracture, bone mineral density loss, metabolic bone loss, osteogenesis imperfecta, osteomalacia, osteonecrosis, osteohalsiteresis, spondyloschisis, and achondroplasia.

In an embodiment, calcium ions are further contained in the medicament for treating or preventing bone-related diseases and/or conditions to administering calcium ions to the subject in need thereof, and the calcium ions and the oligopeptide generate a synergistic effect.

In an embodiment of the oligopeptide composition, the composition is in a form of emulsion, tablet, capsule, granule, pill, syrup, powder, paste, oral liquid or injection.

### BRIEF DESCRIPTION OF THE DRAWINGS

The patent or application file contains at least one drawing executed in color. Copies of this patent or patent application publication with color drawing(s) will be provided by the Office upon request and payment of the necessary fee.
FIGs. 1A and 1B are the collision induced dissociation (CID) spectra of the active ingredients of the present disclosure.
FIGs. 2A-2D show the results of proliferation of osteoblastic cells at 24 hours after administration of an oligopeptide including threonine-serine-lysine-tyrosine-arginine (Thr-Ser-Lys-Tyr-Arg, or referred as TSKYR), threonine-serine-lysine-tyrosine (Thr-Ser-Lys-Tyr, or referred as TSKY), TSK and YR, etc., respectively.
FIGs. 3A-3D show the results of effects on osteoblastic cells proliferation at 24 hours after administration of an oligopeptide including TSKYR, TSKY, TSK and YR, in the presence and absence of calcium ions, respectively.
FIGs. 4A-4E are Von Kossa stained images showing effects on calcific nodules of osteoblastic cells in the control group (no administration of oligopeptide) and at 21 days after administration of an oligopeptide including TSKYR, TSKY, TSK and YR, respectively.
FIGs. 5A-5D show the results of effects on cartilage cells proliferation at 24 hours after administration of an oligopeptide including TSKYR, TSKY, TSK and YR, respectively.
FIG. 6A shows the result of alkaline phosphatase activity in osteoblastic cells at 2 days after administering CaCl₂ alone; FIG. 6B shows the results of alkaline phosphatase activities in osteoblastic cells at 2 days after administering CaCl₂ in combination with an oligopeptide such as TSKYR, TSKY, TSK and YR.
FIGs. 7A and 7B show the results of aggrecan (ACAN) and type II collagen (COL2A1) gene expression in cartilage cells at 24 hours after administration of an oligopeptide including TSKYR, TSK and YR, respectively.
FIGs. 8A and 8B show the results of RUNX2 and OCN gene expression in osteoblastic cells at 2 hours after administration of an oligopeptide including TSKYR, TSK and YR; FIGs. 8C and 8D show the results of FGFR2, and FGFR3 gene expression in osteoblastic cells at 6 hours after administration of an oligopeptide including TSKYR, TSK and YR, respectively.

### DETAILED DESCRIPTION OF THE PREFERRED EMBODIMENTS

The embodiments of the present disclosure will be illustrated by the following embodiments, and those skilled in the art to which the present disclosure belongs can easily understand the advantages and benefits of the present disclosure from the content disclosed herein. However, the embodiments described herein are not intended to limit the present disclosure which can be practiced or applied by other different implementations, and different alternations or modifications can be made to the details described herein based on different views and applications without departing from the scope of the present disclosure.

In addition, the singular form "a/an" and "the" used herein also comprises the plural form, unless otherwise indicated clearly, and the "or" used herein can be interchangeably used with "and/or".

The numerical value ranges recited herein are inclusive and combinable, any numerical values falling in the numerical value ranges can be used as the upper or the lower limit to derive subranges; for example, the numerical value range of "2 to 10" can be understood as any subrange including 2 as the lower limit and 10 as the upper limit, e.g., subranges such as 2 to 8, 3 to 10, and 3 to 8, etc. Also, all numerical values falling in the range should be included, for example, referred as "2 to 10" of course comprises 2, 3, 4, 5, 6, 7, 8, 9, 10.

The first aspect of the present disclosure provides an oligopeptide comprising 2 to 10 amino acids, wherein the oligopeptide comprises the amino acid sequence of Tyr-Arg (YR) and/or Thr-Ser-Lys (TSK). In some embodiments, the oligopeptide comprises at least one amino acid sequence selected from Thr-Ser-Lys-Tyr-Arg (TSKYR, SEQ ID No. 1), Thr-Ser-Lys-Tyr (TSKY, SEQ ID No. 2), Thr-Ser-Lys (TSK), and Tyr-Arg (YR). In further embodiments, the oligopeptide is Thr-Ser-Lys-Tyr-Arg (TSKYR, SEQ ID No. 1), Thr-Ser-Lys-Tyr (TSKY, SEQ ID No. 2), Thr-Ser-Lys (TSK) or Tyr-Arg (YR).

The oligopeptide of the present disclosure can be obtained by an extraction process, a fermentation process, a hydrolysis process, a synthesis process, a generic recombination engineering process, or a combination thereof.

The term "extraction process" refers to a process by which a substance is separated from a mixture based on the different solubility of the substance in different solvents, wherein the mixture for obtaining the novel peptide of the present disclosure is derived from but not limited to microorganisms, plants or animals. Further, the techniques for separation and/or purification include, but not limited to, separation of a peptide with a specific size by using protein electrophoresis method, liquid chromatography, separation through filter membrane with different pore sizes, and the like, and the techniques are not depicted in detail herein since they are those well known by one skilled in the art to which the present disclosure belongs.

The term "fermentation process" refers to a process performed by subjecting plants, animals or derivatives thereof to a fermentation process with a microorganism to obtain different metabolites, such as peptides, amino acids, etc., and specific metabolites can be further obtained by separation purification techniques, e.g., various bioactive peptides are contained in the product from fermentation of soybeans.

The term "hydrolysis process" refers to a process performed by subjecting microorganisms, plants, animals or derivatives thereof to a hydrolysis procedure with an enzyme or acid to generate specific peptides, e.g., casein phosphopeptides are obtained from casein via hydrolysis with pancreatic enzymes or trypsin.

The term "synthesis process" refers to a process performed by connecting amino acids via a synthetic manner to form a polypeptide, which generally has the following advantages: the alternation of the primary structure of the polypeptide, the addition of a special amino acid, and the modification of terminus of the polypeptide can be conveniently carried out.

Further, the synthetic manner includes a chemical synthesis process and a peptide synthesizer. The chemical synthesis process can be classified into solid phase peptide synthesis process and liquid phase peptide synthesis process, wherein in the liquid phase peptide synthesis process, an extraction must be performed after connection of each amino acid. However, the polypeptide intermediate obtained by the extraction is often a mixture and thus is needed to be subjected to a chromatographic purification step, therefore, for synthesis of a polypeptide with a liquid phase synthesis process, complex extraction and chromatographic purification steps must be involved to yield a product with a high purity. In addition, a solid phase process and a liquid phase process also can be combined for peptide synthesis (see, Bruckdorfer et al., 2004 and references recited therein).

A solid phase synthesis process is a bonding reaction of peptide on solid polymer granules (or a polymeric support). In the process, the N-terminal amino acid of a desired polypeptide is firstly bonded covalently to the polymer granules, then subsequent amino acids are connected in order by a manner of specific bonding, and the polypeptide is finally synthesized. The polymer granules (and the desired polypeptide attached on the polymer granules) can be separated from the reactants and by-products only by washing and filtration at the end of the reaction, since the polymer granules are not soluble in solvents. Therefore, since there is no need for purification of intermediates, the solid phase synthesis process has not only a higher yield but also shortened reaction time, has advantages in synthesis of a polypeptide with long chain, and is the one which finds a broader application range currently.

The term "generic recombination engineering process" refers to a process performed by constructing a nucleic acid expressing a specific protein or polypeptide on an expression vector by a biological technique, then transforming the recombinant vector into a host cell such as Escherichia coli, yeast, Lactobacillus, etc., to enable the recombinant expression vector to express the nucleic acid in the hose cell, thereby obtaining the specific protein.

In some embodiments, the oligopeptide involved in the present disclosure can be obtained by using a hydrolysis process. Specifically, the oligopeptide of the present disclosure can be obtained from enzymatic treatment of deer horn which has been used as a traditional Chinese medicine, and the deer horn is taken from a cervid animal, e.g., sika deer, sambar deer, red deer, etc. The oligopeptide of the present disclosure can also be obtained from enzymatic treatment of a compound traditional Chinese medicine containing deer horn, and the compound traditional Chinese medicine is, e.g., Tortoise and Deer Gelatin which comprises at least both of tortoise plastron and deer horn.

The hydrolysis involves enzymatic treatment. Although the enzyme used has no special limit, the enzyme from a source other than pathogenic microorganisms is preferred taking the use of the resulting oligopeptide in particular health foods or pharmaceutical products. The conditions for the enzymatic treatment, such as time, temperature, concentration, etc., vary according to the enzyme used and can be adjusted based on well-known or commonly used in the art, to achieve the best enzymatic treatment conditions.

The enzyme used for the enzymatic treatment described above has no particular limit in the present disclosure and can be, for example, an enzyme capable of cleaving the peptide bonding in collagen or gelatin, with a proteolytic enzyme or a protease can be generally used. As a specific example, a collagenase, a thiol protease, a serine protease, an acidic protease, a basic protease, a metalloprotease, etc. can be used alone or in a combination of foregoing. As a thiol protease described above, a chymopapain, a papain, a bromelin, and a ficin derived from a plant; and cathepsin, and a calcium-dependent protease derived from an animal can be used. In addition, as the serine protease described above, a trypsin, a cathepsin D, etc., can be used. As the acidic protease described above, a known pepsin, chymotrypsin, etc. can be used.

In some embodiments, the hydrolysis product resulting from the enzymatic treatment described above can be purified by one or a combination of more of the following techniques: recrystallization, ultrafiltration, gel filtration, size exclusion chromatography (SEC), ion exchange chromatography (IEC), high performance liquid chromatography (HPLC), hydrophobic chromatography (HIC), reverse-phase chromatography (RPC), affinity chromatography, thin layer chromatography (TLC), electrophoresis (particularly capillary electrophoresis), solid phase extraction (CSPE), amino acid assay post acid hydrolysis, fast atom bombardment(FAB) mass spectrometry, and matrix-assisted laser desorption ionization (MALDI) or electrospray ionization (ESI) quadrupole flight time (Q-TOF) mass spectrometry.

In the present disclosure, the oligopeptide comprises an oligopeptide with or without chemical modification. The specific means or treatment conditions for chemical modification can be applied to the chemical modification technique for general peptides.

A group of amino acids on a peptide can be chemically modified, e.g., for a hydroxyproline, the hydroxy group can be chemically modified. Several effects can be achieved by the chemical modification, for example, a conversion from weakly acidic to neutral for improving solubility. In particular, chemical modifications such as O-acetylation for the hydroxyl group on hydroxyproline; esterification, amidation, etc. for α-carboxyl group of glycine; polypeptidization, succinylation, maleylation, acetylation, deamination, benzoylation, alkylsulfonylation, allylsulfonylation, dinitrophenylation, trinitrophenylation, carbamylation, phenylcarbamylation, thiolation, etc. for α-amino group of proline can be made. The chemical modifications can be appropriately selected as actual requirement. Also, a particular peptide can be made to be basic by ethylenediamination, spermidination, etc.

The specific methods or treatment conditions for chemical modification can be applied to the chemical modification technique for general peptides. For example, for the chemical modification such as O-acetylation of hydroxyl group of hydroxyproline can be performed by enabling an acetic anhydride to function in an aqueous or a non-aqueous solvent. For the chemical modification of α-carboxyl group of glycine, for example, an esterification can be performed by suspending in methanol and then charging dry hydrogen chloride gas, and an amidation can be performed by enabling carbodiimide to function.

In some embodiments, the oligopeptide of the present disclosure can be obtained by a chemical synthesis process. Particularly, the solid phase synthesis process or the liquid phase synthesis process as described above can be employed, wherein the solid phase synthesis process can be an Fmoc (fluorenyl-methoxy-carbonyl) process and/or a Boc (tert-butoxy-carbonyl) process.

The synthesis of a proline-hydroxyproline-glycine (POG) oligopeptide with a solid phase synthesis process is illustrated in detail. The synthesis can be performed by immobilizing proline on a support (polystyrene) and performing a known solid phase synthesis process using an Fmoc group or a Boc group as the amino protecting group. That is, in a dehydration reaction by using polystyrene polymer gel beads having diameters of about 0.1 mm and surfaces modified with amino group as the solid phase and using diisopropyl carbodiimide (DIC) as a condensing agent, a proline with the amino group protected with an Fmoc (fluorenyl-methoxy-carbonyl) group is (peptide) bonded with hydroxyoroline, and then the resicual hydroxyproline is removed by completely washing the solid phase with a solvent. Thereafter, the protecting group on proline bonded with the solid phase is removed (deprotection) to synthesize a proline-hydroxyproline (PO). In turn, the amino group of hydroxyproline is (peptide) bonded to glycine, to obtain proline-hydroxyproline-glycine (POG).

In some embodiments, the oligopeptide of the present disclosure can promote the proliferation and differentiation of osteoblastic cells and/or cartilage cells. In some embodiments, the oligopeptide of the present disclosure can increase the gene expression of type II collagen (COL2A1), aggrecan (ACAN), RUNX2, OCN, FGFR2, and/or FGFR3. In some embodiments, the oligopeptide of the present disclosure can particularly increase the gene expression of type II collagen and/or aggrecan in cartilage cells and the gene expression ofRUNX2, OCN, FGFR2, and/or FGFR3 in osteoblastic cells.

In some embodiments, the oligopeptide of the present disclosure can exhibit a synergistic effect with calcium ions, especially exhibit a synergistic effect on the proliferation and/or differentiation of osteoblastic cells. The calcium ions can be provided from calcium alginate, calcium carbonate, calcium phosphate, oyster shell calcium, pearl calcium, calcium citrate, calcium lactate, milk calcium, amino acid complexed calcium or the above combination, and can be provided directly. In some embodiments, the calcium ions are provided by tortoise plastron used as a traditional Chinese medicine, which is taken from plastron of Testudines, such as grass tortoise, Ocadia sinensis, Trachemys scripta elegans, etc. The calcium ions of the present disclosure can also be provided as a compound traditional Chinese medicine containing tortoise plastron, and the compound traditional Chinese medicine is, e.g., Tortoise and Deer Gelatin.

The second aspect of the present disclosure provides a use of the oligopeptide in manufacture of a medicament for treating or preventing bone-related diseases and/or conditions, comprising administering a therapeutically effective amount of the oligopeptide to a subject in need thereof. In some embodiments, the bone-related diseases and/or conditions include osteoarthritis, arthritis, osteoporosis, fracture, bone mineral density loss, metabolic bone loss, osteogenesis imperfecta, osteomalacia, osteonecrosis, osteohalsiteresis, spondyloschisis, and achondroplasia, etc.

The term "effective amount" refers to the amount at which a compound or an active ingredient is needed to produce a desired effect.

In some embodiments, the oligopeptide can be administered to a subject in need thereof in a manner of oral or injection administration.

In some embodiments, the oligopeptide can be administered in combination with calcium ions due to the synergistic effect with calcium ions, i.e., the medicament for treating or preventing bone-related diseases and/or conditions contains the oligopeptide and calcium ions.

The third aspect of the present disclosure provides an oligopeptide composition comprising the oligopeptide as described above and a pharmaceutically acceptable carrier. The oligopeptide composition can be used as a pharmaceutical composition.

The term "pharmaceutical composition" comprises an effective amount of the desired compound or active ingredient to generate a specific effect, and in the present disclosure, the oligopeptide is the compound or the active ingredient for achieving health care of bone and/or for treating or preventing bone-related diseases and/or conditions.

As understood by a person skilled in the art to which the present disclosure belongs, the form of a pharmaceutical composition can vary according to the administration mode for resulting a particular effect, such as the form of emulsion, tablet, capsule, granule, pill, syrup, powder, paste, oral solution or injection solution, and the carrier can be a solid, semi-solid or liquid based on the form of the pharmaceutical composition. For example, the carrier includes, but not limited to, sweetener, solubilizer, cosolvent, emulsifier, lubricant, wetting agent, coating material, perfuming agent, buffering agent, plastilizer, surfactant, foaming agent, anti-foamer, thickener, inclusion agent, humectant, absorbent, diluting agent, flocculating agent and anti-flocculating agent, filtration aid or release retardant.

In some embodiments, the oligopeptide composition can be administered in a manner similar to those for the oligopeptide described above and can be administered by oral administration or through injection administration to, for example, a joint cavity or local skin at joint.

The forth aspect of the present disclosure provides a method for treating or preventing bone-related diseases and/or conditions by using the oligopeptide. And, the fifth aspect of the present disclosure provides a use of the oligopeptide in treatment or prevention of bone-related diseases and/or conditions.

In order to further illustrate the embodiments of the present disclosure and effects thereof, examples will be provided for detailed explanation; however, the examples are provided for illustrative purposes only, and any materials, steps, conditions, measurement methods, vocabulary, etc. used are not intended to limit the scope and meaning of the present disclosure and the claims

### EXAMPLES

In the examples of the present disclosure, the Tortoise and Deer Gelatin was the product available from CHUAN FENG TANG Pharmacy Ltd. Co. (Yonggu Tortoise and Deer Gelatin, containing two traditional Chinese medicines, i.e., tortoise plastron (from *Mauremys reevesii*) and deer horn (from *Cervus elaphus*) in composition, Approved No.: R029901, Taoyuan, Taiwan, China). The pepsin was available from Sigma-Aldrich Co. (Catalog No.: P7000, St. Louis, Michigan, USA). The formic acid was available from Honeywell Co. (Charlotte, North Carolina, USA). The acetonitrile (CH₃CN) was available from Spectrum Chemical Co. (New Brunswick, New Jersey, USA). The oligopeptide was provided by Mingxin Biotechnology Co., LTD (Taipei, Taiwan, China). The human osteoblastic cells hFOB1.19 were available from American Type Culture Collection (ATCC). The human joint cartilage cells C20A4 were available from Merck & Co., Inc. (MWECK, Darmstadt, Germany). TRACP & ALP detection kit was purchased from Takara Bio. Inc. (Shiga, Japan).

### Preparation of oligopeptides

The peptides having the amino acid sequence shown in SEQ ID No.1 were prepared by an Fmoc solid phase synthesis method and the peptide sequences were identified as Thr-Ser-Lys-Tyr-Arg (TSKYR, SEQ ID NO.1), Thr-Ser-Lys-Tyr (TSKY, SEQ ID NO.2), Thr-Ser-Lys (TSK), and Tyr-Arg (YR).

### Cells culture methods

hFOB1.19 cells were cultured in a 1:1 mixed Dulbecco's Modified Eagle's Medium/Ham's F12 Medium (DMEM/F-12 culture medium) containing 2.5 mM L-glutamine (phenol red free), 0.3 mg/ml geneticin (G418) and 10% fetal bovine serum (FBS). The cells were incubated under 5% CO₂ air concentration at 34°C. C20A4 cells were cultured in a DMEM high glucose medium containing 10% FBS at 5% CO₂ air concentration at 37°C.

### Measurement of proliferation

In order to evaluate the effect of the oligopeptide on proliferation of cells, hFOB1.19 or C20A4 cells were cultured at 4×10⁴ cells/well in a 24-well plate, and were treated with the oligopeptides at different concentrations for 24 hours in the presence or absence of CaCl₂. Then, the cells were treated with trypsin for 2 minutes and counted manually with a blood cell counter.

### Detection of alkaline phosphatase (ALP) activity

The activity of alkaline phosphatase was measured by a TRACP & ALP detection kit. hFOB1.19 cells were cultured at 2×10⁴ cells/well in a 96-well plate for 2 days. After removing the supernatant of the culture, the cells were resuspended in physiological saline. To each well 50 µl of the extract solution was added, and incubated at room temperature for 5 minutes. Then, 50 µl of the matrix solution was added to each well and reacted at 37°C for 60 minutes. Finally, 50 µl of 0.5N NaOH was added to each well to terminate the reaction. After formation of color, the solution was measured for the absorbance at 405 nm.

### Quantitation of bone nodules

Osteoblastic cells hFOB1.19 were cultured in a culturing medium containing 10 nM dexamethasone, 50 µg/ml ascorbic acid, 10 mM β-glycerophosphate and oligopeptide. After incubating for 21 days, the celled were fixed with 4% paraformaldehyde and stained with 5% AgNO₃ in a dark room for 30 minutes. Then, the cells were rinsed with distilled water and exposed to ultraviolet on a sterilized bench for 1 hour. To the reaction 5% sodium thiosulfate was added, after reacting for 2 minutes, sodium thiosulfate was removed, then 0.1% nuclear fast red-aluminum sulfate solution was added to absorb the dye for 5 minutes. Then, calcific nodules were observed and photographed under a microscope. The number, average area and density of all calcific nodules were quantified by using the Image J software.

### Example 1: Separation of oligopeptide from Tortoise and Deer Gelatin by a hydrolysis process and a high performance liquid chromatography process

1 Gram of Tortoise and Deer Gelatin was boiled in water for at least 1 hour and was disintegrate into pieces and dissolved during the process, and could be cut into small pieces manually in order to make it dissolve easily. Additionally, it could be stirred in an intermittent manner to for a uniform solution. After boiling for at least 1 hour, it was cooled to room temperature, and the pH of the solution was adjusted to 1.8 by adding 1N hydrochloric acid. Then, 10 activity units of protease: pepsin was added to the solution and the reaction was performed in a water bath at 37°C for 1 hour. The pH of the solution was adjusted to 7.0 by adding 1N NaOH, to stop the proteolytic reaction. Then, 2 mL of the pepsin-digested sample solution was filtered through a 0.22 µm syringe filter and injected directly into an HPLC column for the first rough separation and activity assay.

At room temperature, the digested peptides were separated by high performance liquid chromatography (L-7000 HPLC, Hitachi Co., Tokyo, Japan) system equipped with a quaternary gradient pump and a photo diode array detector. The chromatography data were collected and processed by the Hitachi HSM software using an analysis column µBondapak^{™} C18 Column, 125Å, 10µm, 3.9×300 mm (Waters Co., Milford, Massachusetts, USA).

The pepsin-digested sample was separated by a gradient elution with Solvent A (0.1% formic acid) and Solvent B (90% CH₃CN with 0.1% formic aid) at a flow rate of 1 mL/min. The wavelengths for ultraviolet detection were 210, 254 and 280 nm. During the HPLC separation, each 1 mL eluent was collected in one tube. The first fraction eluent (0 to 22 minutes) having activity was collected and frozen-dried with a recovery rate of 30.8%. The dried powder was combined and dissolved in water to a final volume of 2 mL. Then, the sample was subjected to LC-MS/MS analysis (the second separation) with the Q Exactive^{™} mass spectrometer (Thermo Scientific, Waltham, Massachusetts, USA) operated in the positive electrospray ionization mode using Solvent A (water), Solvent B (water with 0.1% formic acid) and Solvent C (90% CH₃CN with 0.1% formic acid) at a flow rate of 1 mL/min. The first and second separation processes were shown in Table 1.

**Table 1**

| First separation | | Second separation (LC-MS analysis) | | |
|---|---|---|---|---|
| Time (min) | Eluent (B%) | Time (min) | Eluent (B%) | Eluent (C%) |
| 0-15 | 0 | 0-10 | 0 | 0 |
| 15-20 | 0-15 | 10-25 | 0-100 | 0 |
| 20-40 | 15 | 25-30 | 100 | 80-100 |
| 40-45 | 15-20 | 30-40 | 100-0 | 0-100 |
| 45-75 | 20 | 40-45 | | 100 |
| 75-80 | 20-100 | 45-60 | | 100-0 |
| 80-90 | 100 | | | |
| 90-100 | 100-0 | | | |

The peptides were identified in a smoothed base peak chromatograms (BPC) by the Proteome Discoverer 1.4 software (Thermo Scientific, Waltham, Massachusetts, USA), and bioactive peptides were identified by Uniprot (https://www.uniprot.org/) Cervus elaphus (red deer) proteins database, wherein the database comprises 20274 sequences and 7175222 residues.

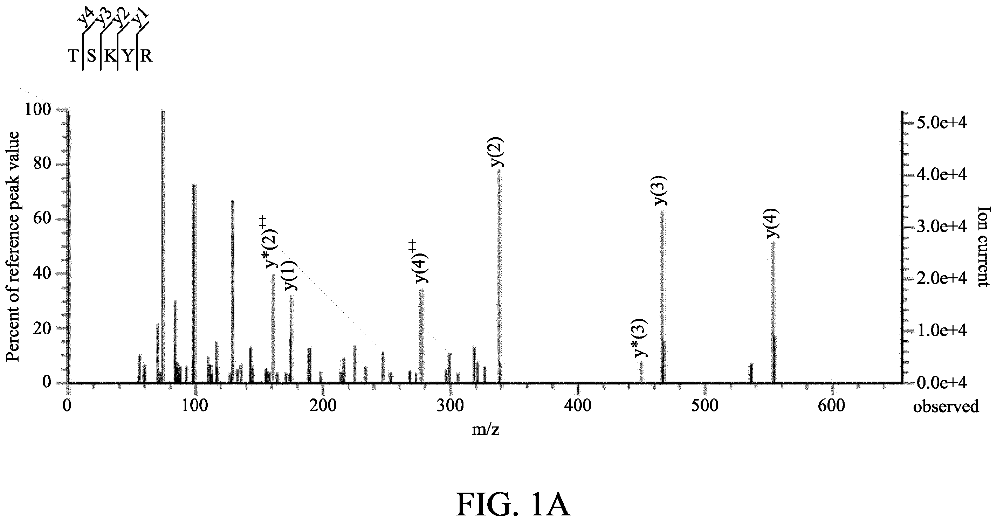

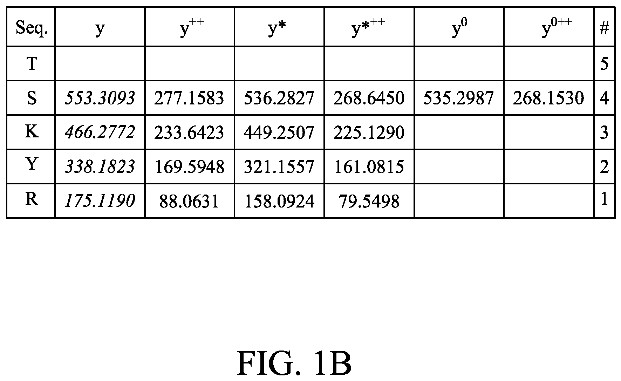

During the second separation process, a single absorption peak having activity at m/z = 327.6819 could be identified, wherein the found molecular weight and calculated molecular weight were 653.3493 and 653.3497 (mass error = -0.47 ppm), respectively. The obtained active ingredients can be determined by MS/MS fragments, the collision-induced dissociation (CID) spectra of which are shown in FIGs. 1A and 1B. The fragments can be corresponding to the genome database of *Cervus elaphus* (red deer), and the following seven proteins in the database contain pentapeptide TSKYR: hemoglobin subunit α (Accession No.: XP_043771628), katanin p60 ATPase containing subunit A1 (Accession No.: XP_043744346), katanin p60 ATPase containing subunit A-like 1 isotype X1 (Accession No.: XP_XP_043747873), katanin p60 ATPase containing subunit A-like 1 isotype X2 (Accession No.: XP_043747878), zinc finger protein 131 isotype X1 (Accession No.: XP_043743469), 60S ribosomal protein L5-like (Accession No.: XP_043726464), piggyBac transposable element-derived protein 1 (Accession No.: XP_043764083). The results showed that the active ingredient was a pentapeptide having the amino acid sequence of TSKYR, as shown by SEQ ID NO.1.

### Example 2: Manufacture of the oligopeptide by synthesis

The active ingredient identified in Example 1 was the pentapeptide TSKYR which could be further degraded into smaller fragments since the pentapeptide TSKYR would be digested by various other proteases during the digestion in intestinal tract of human body. In the present example, in addition to the synthesis of the pentapeptide TSKYR (labeled as P5) by a synthesis process, the following four peptides were synthesized and studied for respective activity thereof: tetrapeptide TSKY (SEQ ID NO.2), tripeptide TSK and dipeptide YR (labeled as P4, P3, and P2, respectively).

### Example 3: Measurements of the stimulatory effects of the oligopeptides on proliferation of osteoblastic cells

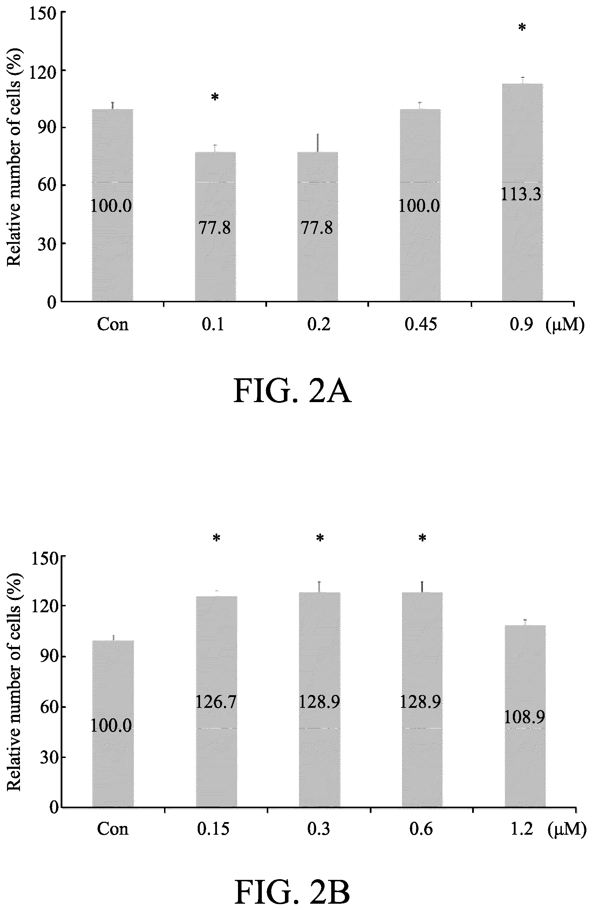

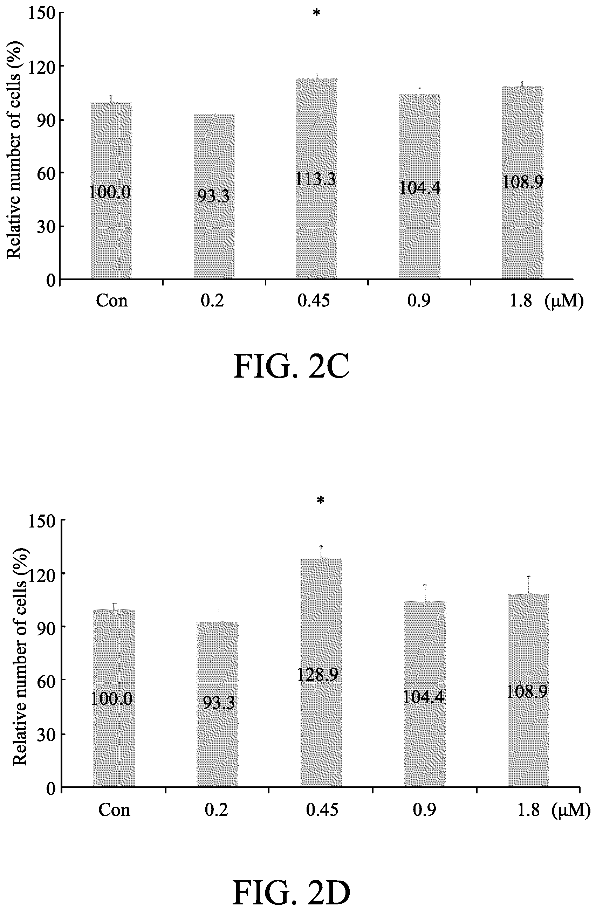

The four oligopeptides, i.e., the pentapeptide TSKYR, the tetrapeptide TSKY, the tripeptide TSK and the dipeptide YR, were assayed for their stimulatory effects on the proliferation of human osteoblastic cells hFOB1.19. After treating human osteoblastic cells hFOB1.19 with the four oligopeptide described above for 24 hours, the number of cells were counted manually to evaluated the stimulatory effects of the four oligopeptides on proliferation of the hFOB1.19 cells. As shown in FIGs. 2A-2D, during the 24 hours administration of the oligopeptides, the four oligopeptides, TSKYR, TSKY, TSK, and YR had best work concentrations of 0.9, 0.6, 0.45, and 0.45 µM, respectively. (In FIGs. 2A-2D, three separate experiments were performed for each group, and the data were expressed by mean ± SD. Statistical significance: *P < 0.05, in respect to the Control groups (A-D). By one-tailed test analysis, *P < 0.05 and * P < 0.005 among groups with different oligopeptide concentrations).

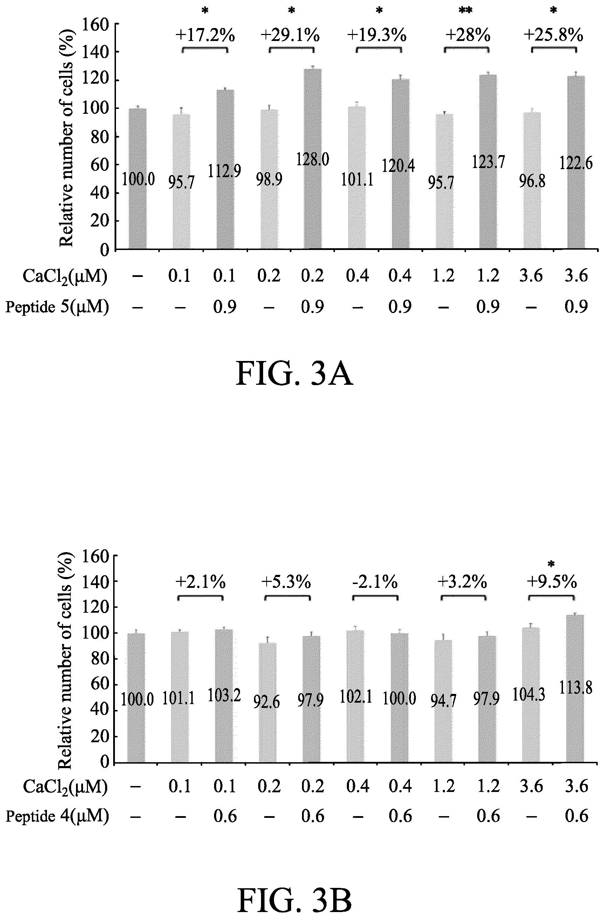

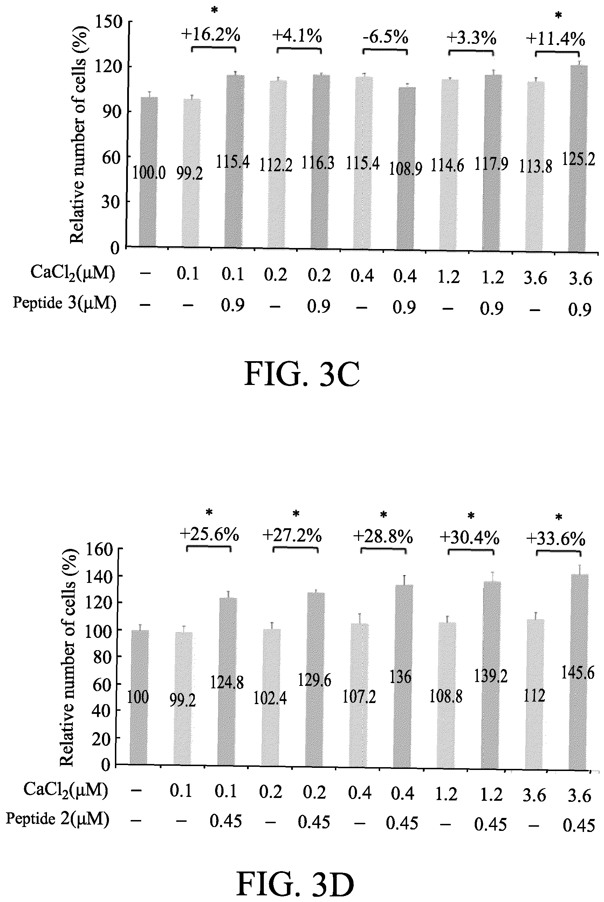

Further, the cells proliferation experiments were performed and compared in the presence and absence of 0.1, 0.2, 0.4, 1.2, and 3.6 µM CaCl₂. As shown in FIGs. 3A-3D, after administering TSKYR, TSK, and YR to human osteoblastic cells hFOB1.19, the number of cells was increased by 29.1% (with 0.2 µM CaCl₂), 9.5% (with 3.6 µM CaCl₂), 16.2% (with 0.1 µM CaCl₂) and 33.6% (with 3.6 µM CaCl₂). The results described above demonstrated the synergistic effects between of calcium ions and the oligopeptide, and the stimulatory effects on proliferation of human osteoblastic cells hFOB1.19 had density ranking of YR > TSKYR > TSK > TSKY. (In FIGs. 3A-3D, three separate experiments were performed for each group, and the data were expressed by mean ± SD. Statistical significance: *P < 0.05, in respect to the Control groups (A-D). By one-tailed test analysis, *P < 0.05 and * P < 0.005, between the group of administering the oligopeptide and CaCl₂ and the group of administering CaCl₂ alone).

### Example 4: Effect of the oligopeptide on the formation of calcific nodules

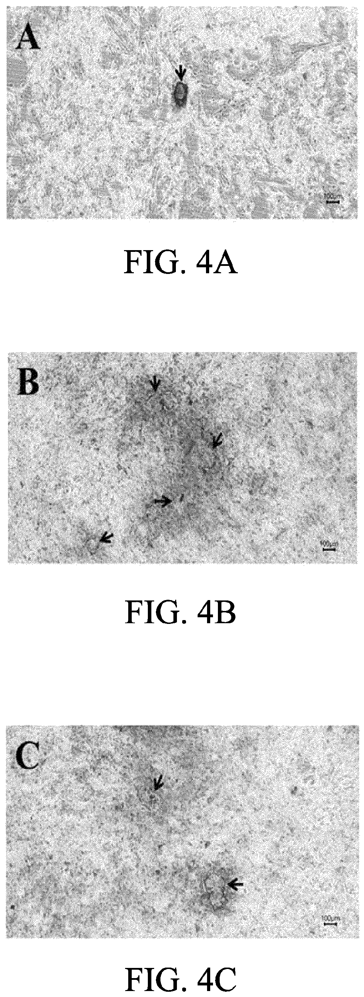

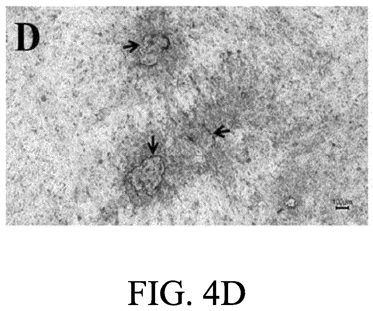

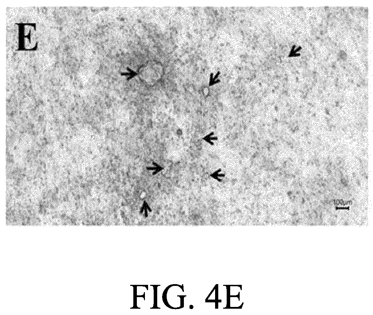

The total number of calcific nodules is the specific indication for mature of the osteoblastic cells. After administering 0.45 M oligopeptide for 21 days, the calcific nodules of human osteoblastic cells hFOB1.19 were stained by Von Kossa staining method. As shown in FIGs. 4A-4E, calcific nodules were the brown to black spots indicated by arrows, and the background was shown in pink. Compared with the control group A shown in FIG 4A, a significantly increased number of calcific nodules was observed in osteoblastic cells hFOB1.19 administered with the oligopeptide.

In addition, as shown in Table 2A, administration of oligopeptide TSKYR, TSKY, TSK, and YR increased the number of calcific nodules by 3.1 folds, 2.3 folds, 2.4 folds and 5.1 folds, wherein the oligopeptide YR had the most effect on promoting the deposition of calcific nodules. As shown in Tables 2B and 2C, the oligopeptide TSK increased the area of nodules by 31.6 folds and increased the density of nodule by 55.2 folds. It can be seen that the oligopeptide TSK was the most effective one for improving the densities and sizes of nodules.

**Table 2A**

| Number of nodules | Control | TSKYR | TSKY | TSK | YR |
|---|---|---|---|---|---|
| Avg. Number | 4.0±0 | 12.5±0.7 | 9.0±1.4 | 9.5±0.7 | 20.5±0.7 |
| Folds | 1.0±0 | 3.1± 0.2 | 2.3±0.4 | 2.4±0.2 | 5.1±0.2 |

**Table 2B**

| Area of nodules | Control | TSKYR | TSKY | TSK | YR |
|---|---|---|---|---|---|
| Avg. Area (Arbitrary Unit (AU)) | 15432 | 19647 | 17276 | 487194 | 17389 |
| Folds | 1.0 | 1.3 | 1.1 | 31.6 | 1.1 |

**Table 2C**

| Nodule density | Control | TSKYR | TSKY | TSK | YR |
|---|---|---|---|---|---|
| Avg. Density (Arbitrary Unit (AU)) | 1796078 | 3130812 | 2487120 | 99092606 | 1950350 |
| Folds | 1.0 | 1.7 | 1.4 | 55.2 | 1.1 |

### Example 5: Measurements of the stimulatory effects of the oligopeptides on proliferation of cartilage cells

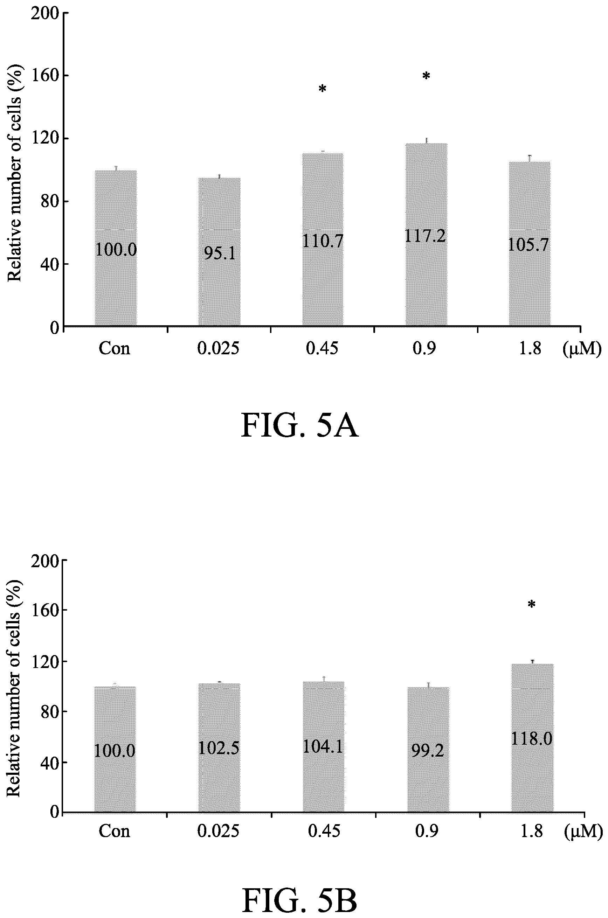

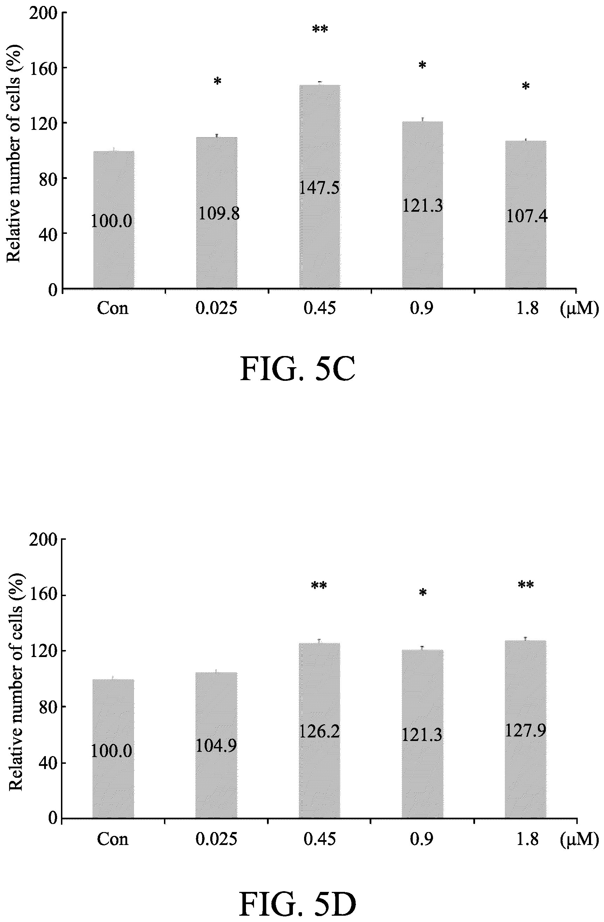

Four oligopeptides at different concentrations were used to treat cartilage cells C20A4 for 24 hours, to assay the stimulatory effect of the oligopeptides on proliferation of human cartilage cells C20A4. The results were shown in FIGs. 5A-5D, which showed that the TSKYR, TSKY, TSK, and YR achieved the highest stimulatory effects on proliferation of human cartilage cells C20A4 at the concentrations of 0.9 µM, 1.8 µM, 0.45 µM, and 1.8 µM, respectively. Treating with TSKYR, TSKY, TSK, and YR increased the numbers of cartilage cells C20A4 by 17.2%, 18.0%, 47.5%, and 27.9%, respectively. Accordingly, it was observed that the oligopeptides stimulated the proliferation of human cartilage cells C20A4 at intensity ranking of TSK > YR > TSKY > TSKYR. (In FIGs. 5A-5D, three separate experiments were performed for each group, and the data were expressed by mean ± SD. Statistical significance: by one-tailed test, *P < 0.05, in respect to the Control groups (A-D).

### Example 6: Measurement results of alkaline phosphatase activities

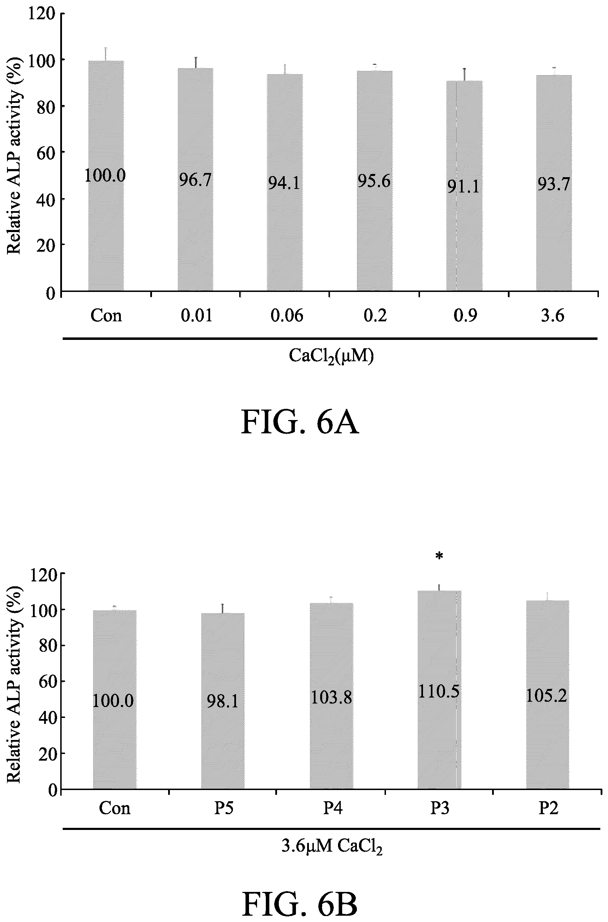

Alkaline phosphatase is present in bone at a high level, and frequently considered as the indicator for differentiation of osteoblastic cells and growth of bone. In Example 6, human osteoblastic cells hFOB1.19 was not treated with the oligopeptide at the condition of different concentrations of CaCl₂, and was treated with the four oligopeptides (0.45 µM) described above at the condition of 3.6 µM CaCl₂ for 2 days, to detect the ALP activities in human osteoblastic cells hFOB1.19. As shown in FIGs. 6A and 6B, under the conditions of various concentrations of CaCl₂ such as 0.01 µM, 0.06 µM, 0.2 µM, 0.9 µM, and 3.6 µM, in the human osteoblastic cells hFOB1.19 which were not treated with the oligopeptides, the ALP activities had no significant change. In contrast, after treating with 0.45 µM of the tetrapeptide, the tripeptide and the dipeptide for 2 days under the condition of 3.6µM CaCl₂, the ALP activities in the osteoblastic cells increased by 3.8%, 10.5% and 5.2%, respectively. Therefore, the results described above showed that calcium ions and the oligopeptides had synergistic effect in promoting differentiation of osteoblastic cells, growth of bone, and proliferation or osteoblastic cells. (In FIGs. 6A and 6B, three separate experiments were performed for each group, and the data were expressed by mean ± SD. Statistical significance: by one-tailed test, *P < 0.05, in respect to the Control groups (A-D).

### Example 7: Effects of the oligopeptide on the gene expressions of aggrecan (ACAN), type II collagen (COL2A1) in human cartilage cells

In human cartilage, type II collagen is one of the main components of extracellular matrix thereof and forms a fiber network; and aggrecan, the dominant proteoglycan in cartilage, not only fill the space among the extracellular fiber network in cartilage along with hyaluronan, but also imparts mechanically press resistance to the cartilage due to the excellent water-retaining property and elasticity. Therefore, in the example, human cartilage cells C20A4 were treated with three oligopeptides: TSKYR (pentapeptide, P5), TSK (tripeptide, P3), and (dipeptide, P2) and gene expressions of type II collagen and aggrecan in the cartilage cells were measured by qPCR, wherein the gene expressions of aggrecan and type II collagen were measured after treating with 0.9 µM of the pentapeptide, 0.45 µM of the tripeptide and 1.8 µM of the dipeptide for 24 hours.

In qPCR, the forwards and reverse primers designed for human type II collagen (COL2A1), aggrecan (ACAN) genes and for human β-actin (ACTB) gene as the control group were as shown in Table 3, below.

**Table 3**

| **Gene** | **Primer sequence** | **Note** |
|---|---|---|
| ACAN | F: CACCTCCCCAACAGATGCTT | SEQ ID No. 3 |
| | R: GGTACTTGTTCCAGCCCTCC | SEQ ID No. 4 |
| COL2A1 | F: TGCTGCCCAGATGGCTGGAGGA | SEQ ID No. 5 |
| | R: TGCCTTGAAATCCTTGAGGCCC | SEQ ID No. 6 |
| β-actin (ACTB) | F: AGAGCTACGAGCTGCCTGAC | SEQ ID No. 7 |
| | R: AGCACTGTGTTGGCGTACA | SEQ ID No. 8 |

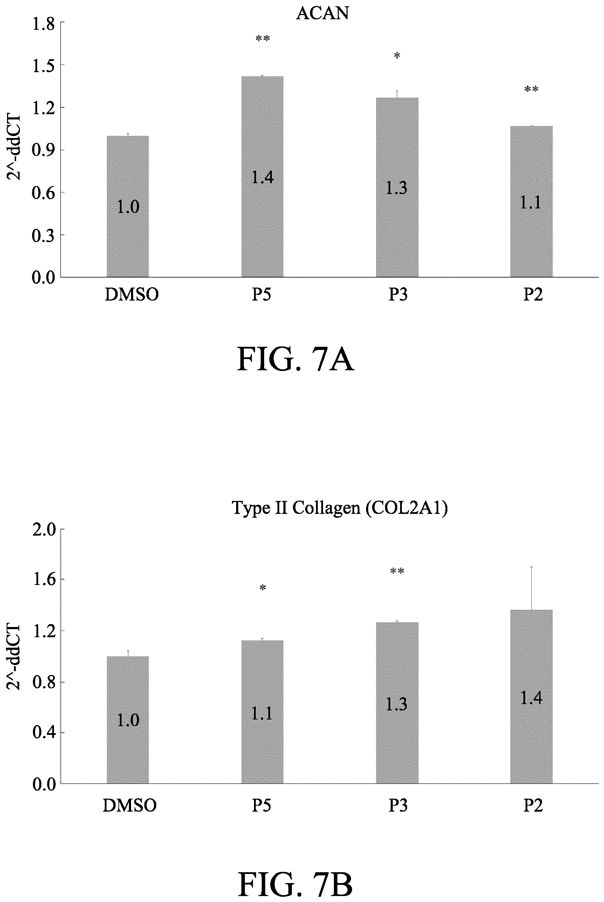

The gene expressions were shown in FIGs. 7A and 7B. The results showed that the cartilage cells treated with the oligopeptides of the present disclosure exhibited significantly increased gene expressions of type II collagen and aggrecan, compared to the cartilage cells which were not treated with the oligopeptide of the present disclosure.

### Example 8: Effects of oligopeptides on gene expressions of RUNX2, OCN, FGFR2, and FGFR3 in human osteoblastic cells

In the example, human osteoblastic cells hFOB1.19 were treated with three oligopeptides (TSKYR(P5), TSK(P3), and YR(P2)) and gene expressions of RUNX2, OCN, FGFR2, and FGFR3 in the osteoblastic cells were measured by qPCR, wherein the RUNX2 and OCN gene expressions were measured after treating with 0.9 µM of the pentapeptide, 0.45 µM of the tripeptide and 0.45 µM of the dipeptide for 2 hours, and the FGFR2, and FGFR3 gene expressions were measured after treating for 6 hours.

In the qPCR, the forward and reverse primers designed for human RUNX2, OCN, FGFR2, and FGFR3 genes had the sequences as shown in Table 4 below.

**Table 4**

| **Gene** | **Primer sequence** | **Note** |
|---|---|---|
| RUNX2 | F: TACTTCGTCAGCATCCTATCA | SEQ ID No. 9 |
| | R: TTCCGTCAGCGTCAACAC | SEQ ID No. 10 |
| OCN | F: GAGGGCAATAAGGTAGTGAA | SEQ ID No. 11 |
| | R: CATAGATGCGTTTGTAGGC | SEQ ID No. 12 |
| FGFR2 | F: GAGAAGGAGATCACGGCTTCC | SEQ ID No. 13 |
| | R: AAGTCTGGCTTCTTGGTCGT | SEQ ID No. 14 |
| FGFR3 | F: GCCTCCTCGGAGTCCTTG | SEQ ID No. 15 |
| | R: CGAAGACCAACTGCTCGTG | SEQ ID No. 16 |

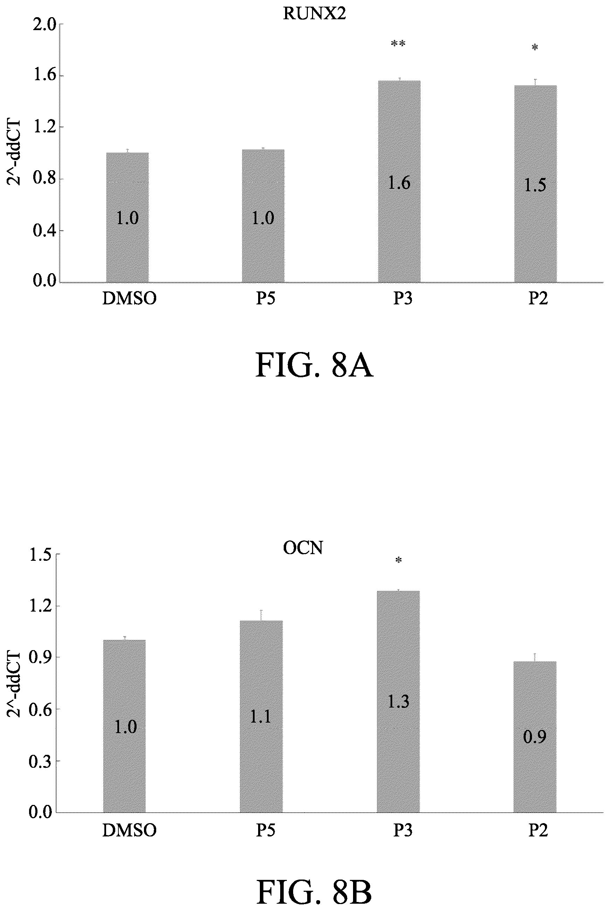

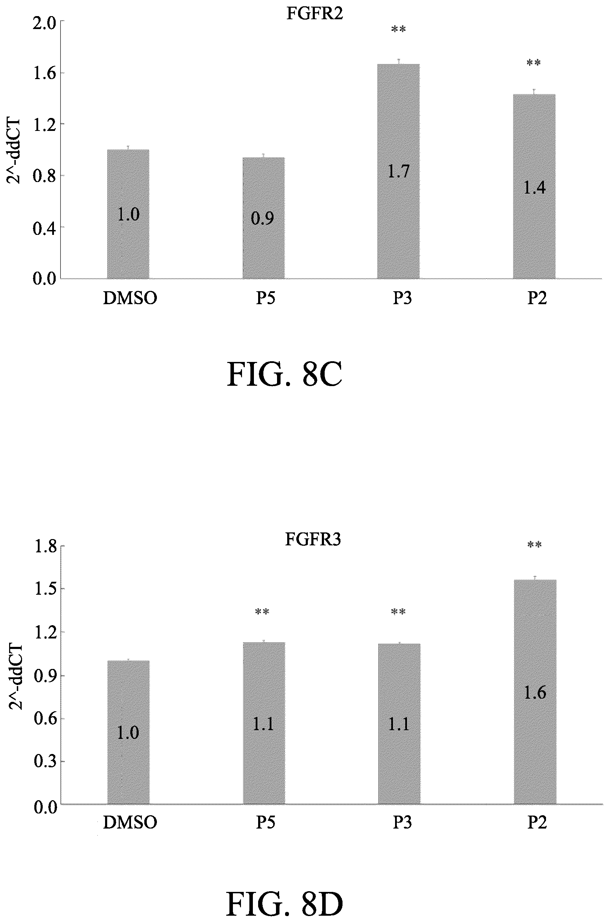

The gene expressions were shown in FIGs. 8A-8D, the osteoblastic cells treated with the oligopeptide of the present disclosure exhibited significantly increased RUNX2, OCN, FGFR2, and FGFR3 gene expressions, compared to the osteoblastic cells which were not treated with the oligopeptide of the present disclosure.

In conclusion, all of the four oligopeptides of the present disclosure, TSKYR, TSKY, TSK and YR, had stimulatory effects on proliferation of human cartilage cells and osteoblastic cells. In addition, with the synergistic effect of the oligopeptide can calcium ions, the number of calcific nodules of osteoblastic cells could be increased, thereby improving the bioavailability of calcium by human body. The experimental results above showed that the synthetic oligopeptide provided in the present disclosure exhibited potency in the treatment or prevention of bone-related diseases, such as osteoporosis, arthrosis deformans, etc.

The examples described above is only for illustrate the technical principle, features and efficacies and is not for limiting the scope which can be practiced by the present disclosure, and one skilled in the art to which the present disclosure belongs can make modifications and alternations to the examples described above without departing from the scope of the present disclosure. However, the equivalent modifications and alternations made based on the teaching of the present disclosure should be encompassed by the claims, and the range claimed by the present disclosure should be as listed in the claims.

## Claims

1. An isolated or synthetic oligopeptide comprising 2 to 10 amino acids, wherein the oligopeptide comprises an amino acid sequence of Tyr-Arg and/or Thr-Ser-Lys.

2. The oligopeptide of claim 1, wherein the oligopeptide comprises at least one amino acid sequence selected from Thr-Ser-Lys-Tyr-Arg, Thr-Ser-Lys-Tyr, Thr-Ser-Lys, and Tyr-Arg.

3. The oligopeptide of claim 1, wherein the oligopeptide promotes the proliferation and differentiation of osteoblastic cells and/or cartilage cells.

4. The oligopeptide of claim 1, which increases an expression of at least one gene selected from the group consisting of COL2A1, ACAN, RUNX2, OCN, FGFR2, and FGFR3.

5. The oligopeptide of claim 1, which is prepared by a hydrolysis process, an extraction process, a fermentation process, a synthesis process, a generic recombination engineering process, or a combination thereof.

6. The oligopeptide of claim 5, which is prepared by the hydrolysis process of a Traditional Chinese medicine or by the synthesis process directly.

7. Use of the oligopeptide of claim 1 in manufacture of a medicament for treating or preventing bone-related diseases and/or conditions, comprising administering a therapeutically effective amount of the oligopeptide to a subject in need thereof.

8. The use of claim 7, wherein the bone-related diseases and/or conditions include osteoarthritis, bone loss, arthritis, osteoporosis, fracture, bone mineral density loss, metabolic bone loss, osteogenesis imperfecta, osteomalacia, osteonecrosis, osteohalsiteresis, spondyloschisis, and achondroplasia.

9. The use of claim 7, wherein the administering is an oral administration or an injection administration.

10. The use of claim 7, wherein the medicament for treating or preventing bone-related diseases and/or conditions further comprises calcium ions for administering the calcium ions to the subject in need thereof.

11. The use of claim 7, wherein the oligopeptide and calcium ions are synergistic for treating or preventing bone-related diseases and/or conditions.

12. An oligopeptide composition comprising the oligopeptide of claim 1 and a pharmaceutically acceptable carrier.

13. The composition of claim 12, further comprising calcium ions.

14. The composition of claim 12, which is for use in bone care and/or in treatment or prevention of bone-related diseases and/or conditions.

15. The composition of claim 12, which is in a form of emulsion, tablet, capsule, granule, pill, syrup, powder, paste, oral liquid or injection.
